# EUROPEAN PATENT APPLICATION

(11) **EP 4 800 091 A1**
(43) Date of publication of application: **02.09.2026**
(21) Application number: 24901066.1
(22) Date of filing: 04.12.2024
(51) Int. Cl.: C12M 1/26, C12M 1/00

(54) **SAMPLE COLLECTION CONTAINER ASSEMBLY HAVING PIPETTE**

(30) Priority: 05.12.2023 KR 20230174976
(71) Applicant: RM Bioscience Co., Ltd., Seoul 08504 (KR)
(72) Inventor: KIM, Il, Seoul 06298 (KR)
(74) Representative: Nederlandsch Octrooibureau
(86) International application number: PCT/KR2024/019750
(87) International publication number: WO 2025/121880

(57) **Abstract**

The present invention relates to an improved container assembly for picking a free medical treatment formed pipet, which enables obtaining a cell circle with a uniform distribution on a membrane filter to improve examination efficiency and enable accurate examination, and to obtain a cell circle with high cell density.

## Description

### TECHNICAL FIELD

The present invention relates to a container assembly for picking a free medical treatment formed pipet, and more particularly, to an improved container assembly for picking a free medical treatment formed pipet, which is provided with a vortex prevention net so as to obtain a cell circle having a uniform distribution on a membrane filter, thereby improving examination efficiency and enabling accurate examination.

Furthermore, the present invention relates to a container assembly for picking a free medical treatment formed pipet, in which a blocking step is formed in a connecting body so as to obtain a cell circle having a high cell density.

### BACKGROUND ART

Cytological examination is a method for observing collected cells under a microscope to determine whether the collected cells have infection, inflammation, abnormal cells, or cancer cells.

Cytological examination is widely used for diagnosing cervical cancer by observing cells collected from cervical tissues or secretions to identify the presence of abnormal cells, and is applied to clinical examination of sputum, urine, gastric fluid, cerebrospinal fluid, bronchoalveolar lavage fluid, and tissue cell culture.

Cytological examination is mainly performed by fixing cells contained in a collected specimen, collecting the cells, applying them to a slide, and reading them under a microscope.

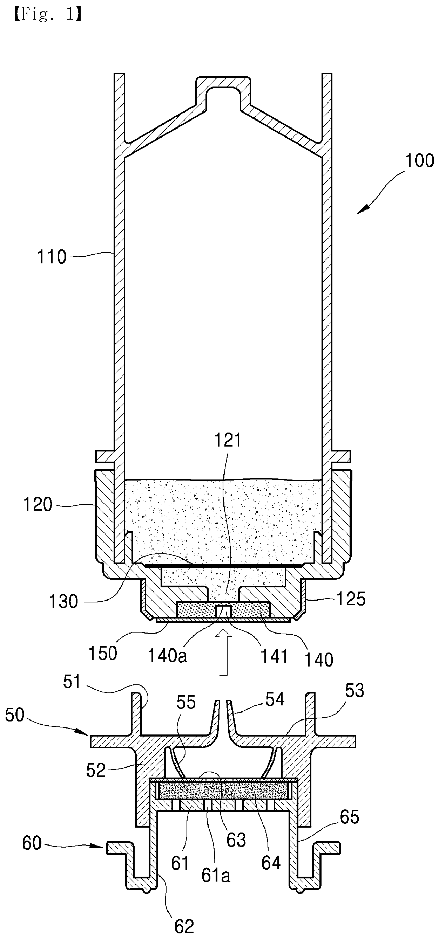

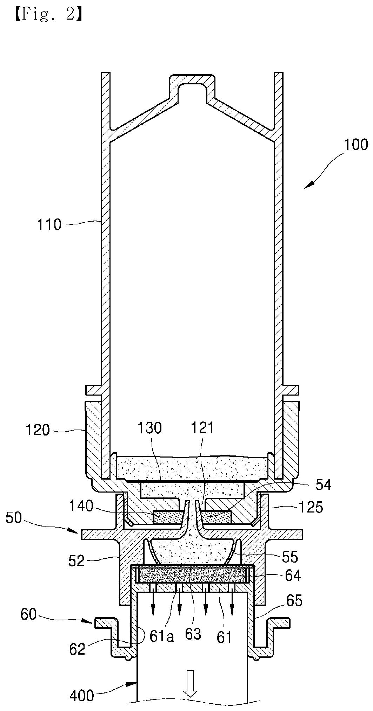

An example of a specimen (cell) collecting container assembly is disclosed in Korean Patent Registration No. 10-1825452, and is shown in FIGS. 1 to 3.

This comprises a container (100) for accommodating a solution and collected cells, a connecting body (50) forming a passage for the solution and cells in the container (100), and a filter fixing body (60) for separating the solution and cells moved through the connecting body (50).

The container (100) comprises a container body (110) with one open end, a cap member (120) detachably coupled to the container body (110) to open/close the opening and having a through-hole (121) formed in the center thereof, and an elastic plate (140) coupled to the through-hole (121).

A filter member (130) for filtering floating matter in the solution is coupled inside the cap member (120).

The connecting body (50) comprises a bottom plate (53) formed such that a container tube (54) entering the inside of the container (100) through the elastic plate (140) protrudes upward from the center, an upper coupling part (51) extending upward from the edge of the bottom plate (53) and detachably coupled to the cap member (120), and a lower coupling part (52) extending downward from the edge of the bottom plate (53).

A guide (55) of an upwardly-flared shape is formed on the lower surface of the bottom plate (53) so that cells guided into the container tube (54) are guided to the center of the membrane filter (63).

The connecting body (50) guides the solution and cells in the container (100) to the lower coupling part (52) side through the container tube (54).

The filter fixing body (60) comprises a body (65) having a cylindrical shape with a middle plate (61) having a suction hole (61a) formed in the middle portion thereof, with an upper end detachably coupled to the lower coupling part (52) and a vacuum suction device (400) coupled to the lower portion of the middle plate (61), and a membrane filter (63) attached to the upper end of the body (65).

A cushion body (64) is provided between the membrane filter (63) and the middle plate (61) to allow the solution to pass through and support the membrane filter (63).

A specimen container assembly configured as above allows the solution and cells in the container (100) to flow into the lower coupling part (52) of the connecting body (50) through the container tube (54) by the suction force of the vacuum suction device (400), and the solution is discharged through the suction hole (61a) of the middle plate (61) while cells are caught on the upper surface of the membrane filter (63).

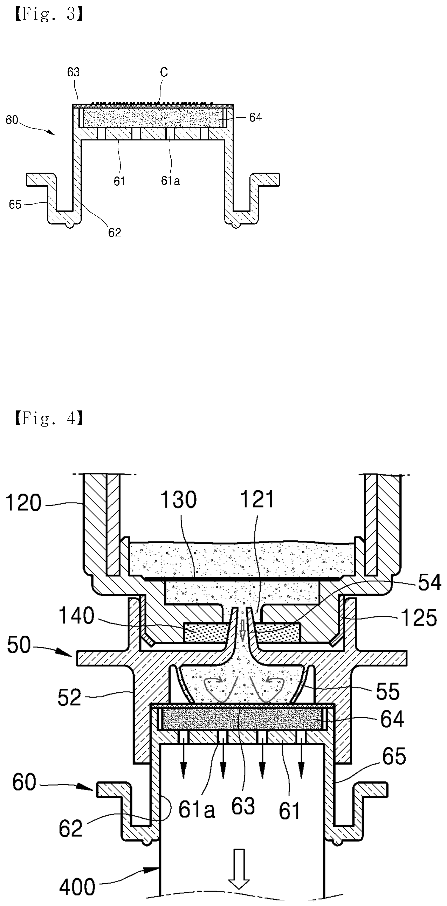

At this time, the cells (C) form a circle in the center of the membrane filter (63) as guided by the guide (55) as shown in FIG. 3.

However, in the specimen container assembly configured as above, when the solution and cells in the container (100) pass through the container tube (54) by the suction force of the vacuum suction device (400), the flow velocity increases and the space expands, causing the solution and cells to form a vortex (refer to arrow in FIG. 4) inside the guide (55), which limits obtaining a cell circle with a uniform distribution on the membrane filter (63).

Furthermore, to minimize the formation of vortex in the solution and cells, the operating speed of the vacuum suction device (400) must be slowed down, which is undesirable as it slows the specimen collection speed.

### SUMMARY OF THE INVENTION

### Technical Problem

The present invention has been created to solve the above problems, and an object of the present invention is to provide an improved container assembly for picking a free medical treatment formed pipet, which minimizes the formation of vortex in the solution and cells, enables obtaining a cell circle with a uniform distribution on a membrane filter, thereby improving examination efficiency and enabling accurate examination.

Another object of the present invention is to provide a container assembly for picking a free medical treatment formed pipet, which enables obtaining a cell circle with high cell density.

### Solution to Problem

The container assembly for picking a free medical treatment formed pipet of the present invention for achieving the above objects,
comprises a container (100) having a container body (110) with one open end, a cap member (120) detachably coupled to the container body (110) to open/close the opening and having a through-hole (121) formed in the center thereof, and a puncturable sealing sheet (150) attached to the cap member (120) to seal the through-hole (121);
a connecting body (50) having a bottom plate (53) formed such that a container tube (54) entering the inside of the container (100) by puncturing the sealing sheet (150) protrudes upward, an upper coupling part (51) extending upward from the edge of the bottom plate (53) and detachably coupled to the cap member (120), and a lower coupling part (52) extending downward from the edge of the bottom plate (53);
a filter fixing body (60) having a cylindrical body (65) with a middle plate (61) having a suction hole (61a) formed in the middle portion thereof, with an upper end detachably coupled to the lower coupling part (52) and a vacuum suction device (400) coupled to the lower portion of the middle plate (61), and a membrane filter (63) attached to the upper end of the body (65) to pass through the solution and filter the cells;
wherein a pressing step (56) is formed on the inner circumferential surface of the lower coupling part (52) against which the edge of the membrane filter (63) is closely supported, and a locking step (55) is formed on the inner circumferential surface of the lower coupling part (52) disposed between the bottom plate (53) and the pressing step (56),
a vortex prevention net (70) is attached to the locking step (55) and through which the solution and cells pass,
so that a first space (58) between the bottom plate (53) and the vortex prevention net (70), and a second space (59) between the vortex prevention net (70) and the membrane filter (63) are formed.

Furthermore, in the container assembly of the present invention, a filter member (130) for filtering floating matter in the solution is further provided inside the cap member (120).

Meanwhile, another container assembly for picking a free medical treatment formed pipet of the present invention for achieving the above objects,
comprises a container (100) having a container body (110) with one open end, a cap member (120) detachably coupled to the container body (110) to open/close the opening and having a through-hole (121) formed in the center thereof, and a puncturable sealing sheet (150) attached to the cap member (120) to seal the through-hole (121);
a connecting body (50) having a bottom plate (53) formed such that a container tube (54) entering the inside of the container (100) by puncturing the sealing sheet (150) protrudes upward, an upper coupling part (51) extending upward from the edge of the bottom plate (53) and detachably coupled to the cap member (120), and a lower coupling part (52) extending downward from the edge of the bottom plate (53);
a filter fixing body (60) having a cylindrical body (65) with an upper end detachably coupled to the lower coupling part (52), an inner middle plate (61) having a suction hole (61a) formed in the middle portion thereof, and a vacuum suction device (400) coupled to the lower portion of the middle plate (61);
a cushion body (64) mounted on the middle plate (61) to pass through the solution;
a membrane filter (63) mounted on the upper surface of the cushion body (64) to pass through the solution and filter the cells;
wherein a pressing step (56) formed on the inner circumferential surface of the lower coupling part (52) to press the edges of the membrane filter (63) and the cushion body (64), a locking step (55) formed on the inner circumferential surface of the lower coupling part (52) disposed between the bottom plate (53) and the pressing step (56), and a blocking step (57) formed on the inner circumferential surface of the lower coupling part (52) between the pressing step (56) and the locking step (55) are provided,
a vortex prevention net (70) is attached to the locking step (55) and through which the solution and cells pass,
so that a first space (58) between the bottom plate (53) and the vortex prevention net (70), and a second space (59) between the vortex prevention net (70) and the membrane filter (63) are formed.

Furthermore, in the container assembly of the present invention, a filter member (130) for filtering floating matter in the solution is further provided inside the cap member (120).

### Advantageous Effects of Invention

First, since the container tube (54) enters the container (100) and extracts cells by suction force, there is an effect of preventing deformation of cells.

Second, the container assembly of the present invention guides the flow in a state where vortex is reduced by the vortex prevention net (70), so that a cell circle with a uniform distribution on the membrane filter (63) can be obtained, thereby improving examination efficiency and enabling accurate examination.

Third, when the connecting body (50) and the filter fixing body (60) are coupled, even if the inclined surface (63a) is formed while the edges of the cushion body (64) and the membrane filter (63) are elastically compressed by the pressing step (56), the blocking step (57) contacts the upper end of the inclined surface (63a), thereby preventing the solution and cells passing through the vortex prevention net (70) from passing through the inclined surface (63a), improving the cell density (C) in the center of the membrane filter (63).

### BRIEF DESCRIPTION OF DRAWINGS

FIG. 1 is a separated cross-sectional view showing a conventional specimen container assembly,
FIG. 2 is an assembly cross-sectional view of the assembly of FIG. 1,
FIG. 3 is a cross-sectional view showing a state in which cells are filtered from the filter fixing body assembled from the assembly of FIG. 2,
FIG. 4 is a view explaining a problem of the container assembly of FIG. 1,
FIG. 5 is a partial separated cross-sectional view showing a container assembly according to a first embodiment of the present invention,
FIG. 6 is a partial separated cross-sectional view of FIG. 5,
FIG. 7 is an assembly cross-sectional view of FIG. 5, explaining the operating state,
FIG. 8 is a partial enlarged view of FIG. 7,
FIGS. 9 and 10 are views for explaining another container assembly,
FIG. 11 is a partial cross-sectional view showing a container assembly according to a second embodiment of the present invention improved over the container assembly of FIG. 9,
FIG. 12 is a plan view showing a state in which cells are caught on the membrane filter,
FIG. 13 is a schematic view showing a state in which cells are applied to glass.

### DESCRIPTION OF EMBODIMENTS

### [First Embodiment]

The container assembly for picking a free medical treatment formed pipet (pipet) according to the first embodiment of the present invention prevents deformation of cells, enables obtaining a cell circle with a uniform distribution on the membrane filter, thereby improving examination efficiency and enabling accurate examination.

In the following description, the same reference numerals and the same names are used for the same parts as in the prior art.

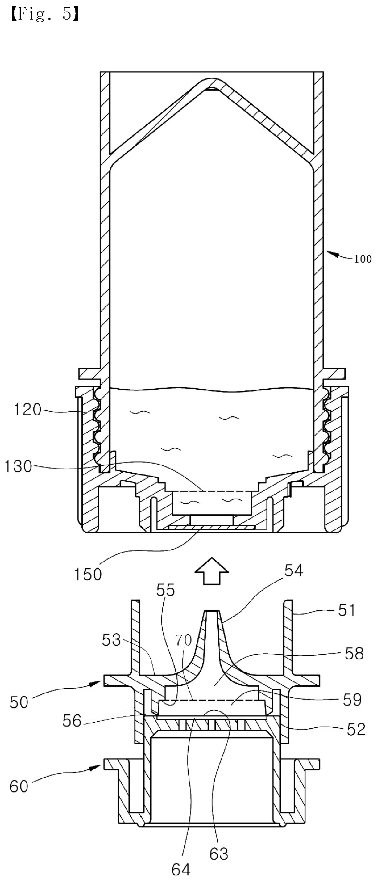

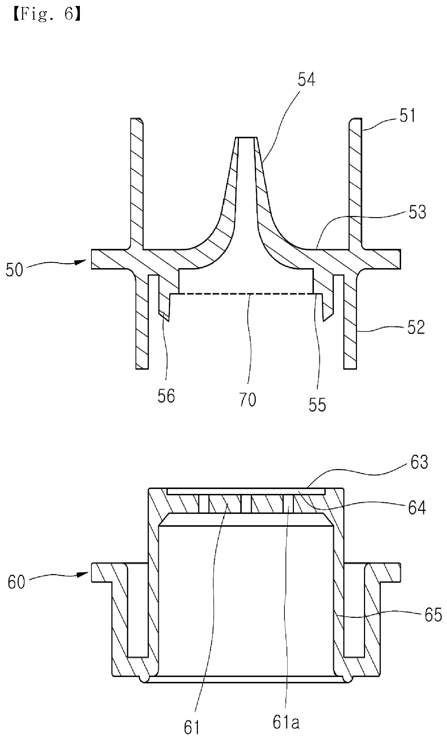

Referring to FIGS. 5 and 6 showing the specimen container assembly according to the first embodiment of the present invention, it comprises a container (100) for accommodating a solution and collected cells, a connecting body (50) forming a passage for the solution and cells in the container (100), and a filter fixing body (60) for separating the solution and cells moved through the connecting body (50).

The container (100) comprises a container body (110) with one open end, a cap member (120) detachably coupled to the container body (110) to open/close the opening and having a through-hole (121) formed in the center thereof, and a puncturable sealing sheet (150) attached to the cap member (120) to seal the through-hole (121).

Furthermore, a filter member (130) for filtering floating matter in the solution is coupled inside the cap member (120).

The connecting body (50) comprises a bottom plate (53) formed such that a container tube (54) entering the inside of the container (100) by puncturing the sealing sheet (150) protrudes upward from the center, an upper coupling part (51) extending upward from the edge of the bottom plate (53) and detachably coupled to the cap member (120), and a lower coupling part (52) extending downward from the edge of the bottom plate (53).

The connecting body (50) guides the solution and cells in the container (100) to the lower coupling part (52) side through the container tube (54).

The filter fixing body (60) comprises a body (65) having a cylindrical shape with a middle plate (61) having a suction hole (61a) formed in the middle portion thereof, with an upper end detachably coupled to the lower coupling part (52) and a vacuum suction device (400) coupled to the lower portion of the middle plate (61), and a membrane filter (63) attached to the upper end of the body (65).

A cushion body (64) for allowing the solution to pass through and supporting the membrane filter (63) is provided between the membrane filter (63) and the middle plate (61).

Meanwhile, a packing (125) made of silicone or rubber material is attached to the outer circumferential surface of the cap member (120) so that airtightness is maintained when the cap member (120) and the upper coupling part (51) are coupled, so that the suction force by the vacuum suction device (400) can be precisely controlled.

Meanwhile, as a characteristic configuration of the first embodiment of the present invention, a pressing step (56) is formed on the inner circumferential surface of the lower coupling part (52) against which the edge of the membrane filter (63) is closely supported, a locking step (55) is formed on the inner circumferential surface of the lower coupling part (52) disposed between the bottom plate (53) and the pressing step (56), and a vortex prevention net (70) whose edge is attached to the locking step (55) and through which the solution and cells pass is provided.

With such a configuration, the container assembly according to the first embodiment of the present invention forms a first space (58) between the bottom plate (53) and the vortex prevention net (70), and a second space (59) between the vortex prevention net (70) and the membrane filter (63).

The method of using the specimen container assembly according to the first embodiment of the present invention configured as above is as follows.

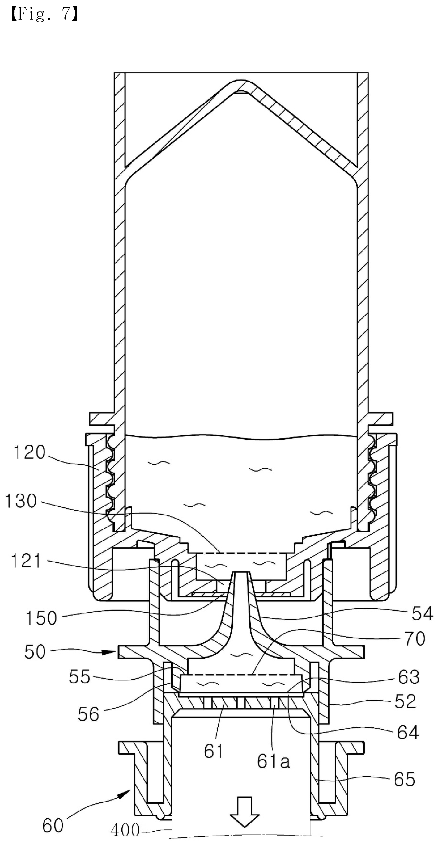

First, the solution and cells are accommodated in the container body (110) and the cap member (120) is coupled, and then the container (100) is inverted so that the cap member (120) is positioned at the bottom as shown in FIG. 7.

At this time, large floating matter is filtered through the filter member (130), and the solution and cells (C) are collected at the lower portion of the cap member (120).

*Next, the connecting body (50) and the filter fixing body (60) are coupled, and in a state where the vacuum suction device (400) is attached to the coupling part (62) of the filter fixing body (60), the sealing sheet (150) is punctured by the container tube (54) to enter inside the container (100).

After this assembly is completed, the solution and cells in the container (100) flow into the lower coupling part (52) of the connecting body (50) through the container tube (54) by the suction force of the vacuum suction device (400), and the solution is discharged downward through the suction hole (61a) of the middle plate (61) while cells are caught on the upper surface of the membrane filter (63).

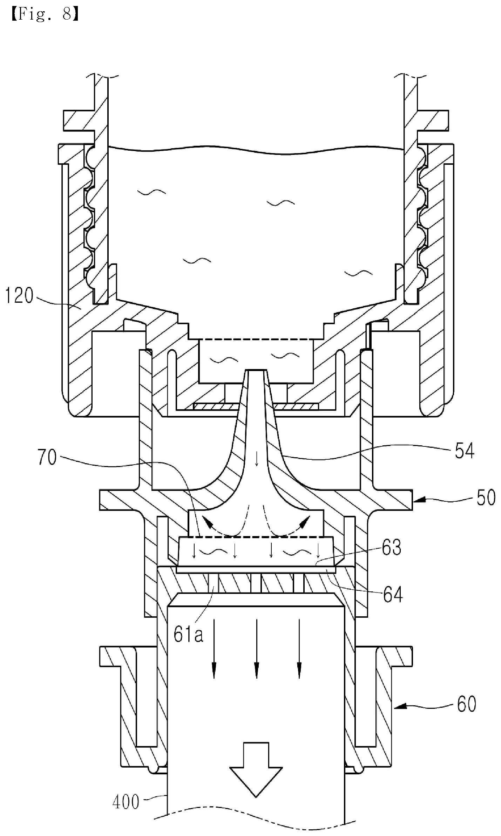

Referring to FIG. 8, when the solution and cells in the container (100) pass through the container tube (54) having a small diameter by the suction force of the vacuum suction device (400), a vortex is formed in the first space (58) where the space expands, and the vortex is reduced by the vortex prevention net (70), so the solution and cells flow into the second space (59).

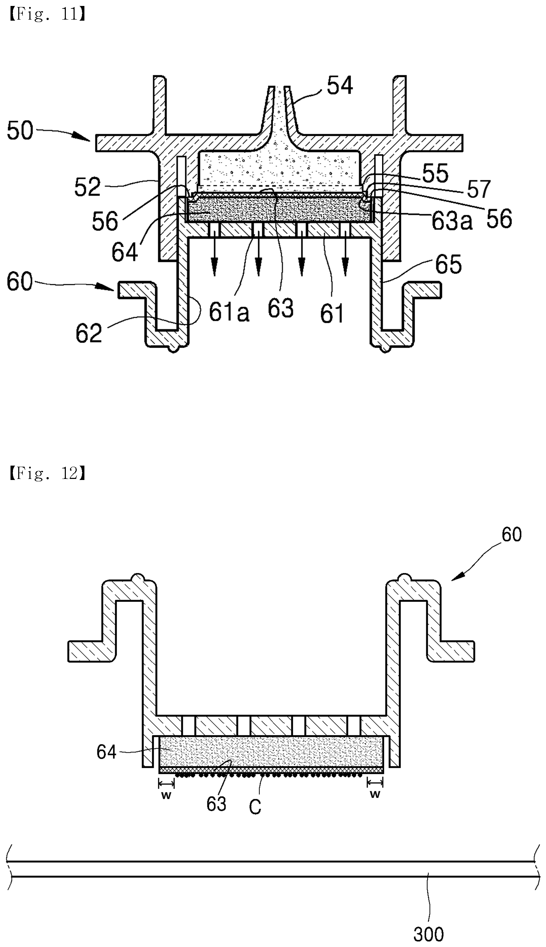

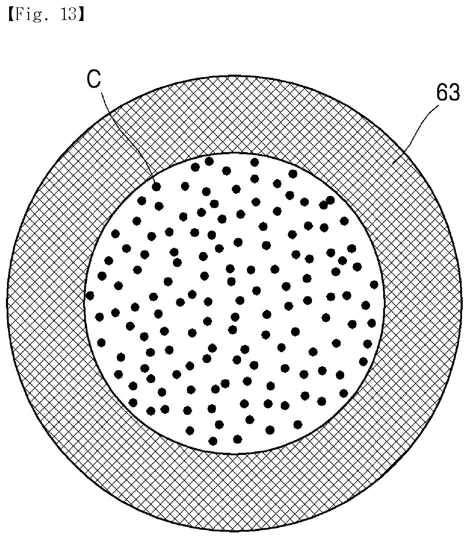

The solution flowing into the second space (59) is discharged downward through the membrane filter (63) and the cushion body (64), and cells (C) are caught on the upper surface of the membrane filter (63) as shown in FIGS. 12 and 13.

At this time, the cells (C) are uniformly stacked on the membrane filter (63) by the vortex-reduced flow, forming a circle.

Thereafter, the operator separates the filter fixing body (60) and brings the membrane filter (63) into contact with the slide (300) as shown in FIG. 13, thereby attaching the specimen cells (C) to the surface of the slide (300).

At this time, the cushion body (64) cushions when the membrane filter (63) contacts the slide (300) so that cells (C) can be easily transferred and attached to the slide (300) and prevents cells (C) from being deformed by the contact pressure against the slide (300).

These specimen cells are attached to the surface of the slide (300) in a circular cell circle form, which has the advantage of improving cell analysis efficiency.

The container assembly according to the first embodiment of the present invention configured as above prevents deformation of cells since the container tube (54) enters the container (100) and extracts cells by suction force.

Furthermore, the container assembly of the present invention guides the flow in a state where vortex is reduced by the vortex prevention net (70), so that a cell circle with a uniform distribution on the membrane filter (63) can be obtained, thereby improving examination efficiency and enabling accurate examination.

### [Second Embodiment]

The container assembly according to the second embodiment of the present invention differs from the container assembly of the first embodiment as follows.

In the container assembly of the first embodiment, the membrane filter (63) is attached to the upper end of the body (65), and a cushion body (64) for supporting the membrane filter (63) is provided between the membrane filter (63) and the middle plate (61).

In the container assembly of the second embodiment, first, the membrane filter (63) is placed on or attached to the upper surface of the cushion body (64), and these cushion body (64) and membrane filter (63) are mounted on the middle plate (61);
second, it differs from the container assembly of the first embodiment in that a blocking step (57) is formed on the inner circumferential surface of the lower coupling part (52) between the pressing step (56) and the locking step (55) of the connecting body (50).

In the following description, the same reference numerals and names as in the first embodiment are used for the same parts as in the first embodiment, and detailed description of the parts described in the first embodiment will be omitted.

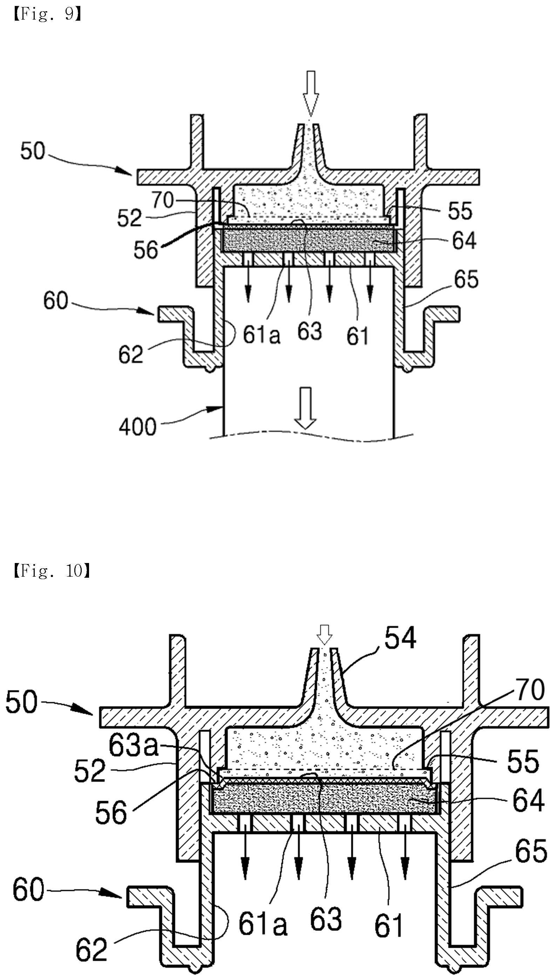

FIGS. 9 and 10 are views for explaining the side effect caused by the configuration in which the cushion body (64) and the membrane filter (63) are mounted on the middle plate (61), and FIG. 11 is a view showing the main part of the container assembly according to the second embodiment of the present invention that addresses such side effects.

Referring to FIGS. 9 and 10, when the cushion body (64) and the membrane filter (63) are mounted on the middle plate (61), when the connecting body (50) and the filter fixing body (60) are coupled, the edges of the cushion body (64) and the membrane filter (63) are elastically compressed by the pressing step (56), forming an inclined surface (63a).

In this case, there is a side effect that the solution and cells passing through the vortex prevention net (70) pass through the inclined surface (63a), causing the cells (C) to be concentrated on the inclined surface (63a) and filtered.

FIG. 11 shows the container assembly according to the second embodiment of the present invention that addresses such side effects. This is a modification of the configuration of the connecting body (50), comprising a pressing step (56) formed on the inner circumferential surface of the lower coupling part (52) to press the edges of the membrane filter (63) and the cushion body (64), a locking step (55) formed on the inner circumferential surface of the lower coupling part (52) disposed between the bottom plate (53) and the pressing step (56), and a blocking step (57) formed on the inner circumferential surface of the lower coupling part (52) between the pressing step (56) and the locking step (55).

With such a configuration, even when the connecting body (50) and the filter fixing body (60) are coupled and the edges of the cushion body (64) and the membrane filter (63) are elastically compressed by the pressing step (56) to form the inclined surface (63a), the blocking step (57) contacts the upper end of the inclined surface (63a), so the second space (59) and the inclined surface (63a) are blocked and separated.

Thereby, the solution and cells passing through the vortex prevention net (70) do not pass through the inclined surface (63a), improving the cell density (C) in the center of the membrane filter (63).

### INDUSTRIAL APPLICABILITY

The container assembly for picking a free medical treatment formed pipet of the present invention is provided with a vortex prevention net so as to obtain a cell circle with a uniform distribution on the membrane filter, thereby improving examination efficiency and enabling accurate examination.

## Claims

1. A container assembly for picking a free medical treatment formed pipet comprising:
a container (100) having a container body (110) with one open end, a cap member (120) detachably coupled to the container body (110) to open/close the opening and having a through-hole (121) formed in the center thereof, and a puncturable sealing sheet (150) attached to the cap member (120) to seal the through-hole (121);
a connecting body (50) having a bottom plate (53) formed such that a container tube (54) entering the inside of the container (100) by puncturing the sealing sheet (150) protrudes upward, an upper coupling part (51) extending upward from the edge of the bottom plate (53) and detachably coupled to the cap member (120), and a lower coupling part (52) extending downward from the edge of the bottom plate (53);
a filter fixing body (60) having a cylindrical body (65) with a middle plate (61) having a suction hole (61a) formed in the middle portion thereof, with an upper end detachably coupled to the lower coupling part (52) and a vacuum suction device (400) coupled to the lower portion of the middle plate (61), and a membrane filter (63) attached to the upper end of the body (65) to pass through the solution and filter the cells;
wherein a pressing step (56) is formed on the inner circumferential surface of the lower coupling part (52) against which the edge of the membrane filter (63) is closely supported, and a locking step (55) is formed on the inner circumferential surface of the lower coupling part (52) disposed between the bottom plate (53) and the pressing step (56),
a vortex prevention net (70) whose edge is attached to the locking step (55) and through which the solution and cells pass is provided,
so that a first space (58) between the bottom plate (53) and the vortex prevention net (70), and a second space (59) between the vortex prevention net (70) and the membrane filter (63) are formed.

2. The container assembly for picking a free medical treatment formed pipet according to Claim 1, wherein a filter member (130) for filtering floating matter in the solution is further provided inside the cap member (120).

3. A container assembly for picking a free medical treatment formed pipet comprising:
a container (100) having a container body (110) with one open end, a cap member (120) detachably coupled to the container body (110) to open/close the opening and having a through-hole (121) formed in the center thereof, and a puncturable sealing sheet (150) attached to the cap member (120) to seal the through-hole (121);
a connecting body (50) having a bottom plate (53) formed such that a container tube (54) entering the inside of the container (100) by puncturing the sealing sheet (150) protrudes upward, an upper coupling part (51) extending upward from the edge of the bottom plate (53) and detachably coupled to the cap member (120), and a lower coupling part (52) extending downward from the edge of the bottom plate (53);
a filter fixing body (60) having a cylindrical body (65) with an upper end detachably coupled to the lower coupling part (52), an inner middle plate (61) having a suction hole (61a) formed in the middle portion thereof, and a vacuum suction device (400) coupled to the lower portion of the middle plate (61);
a cushion body (64) mounted on the middle plate (61) to pass through the solution;
a membrane filter (63) mounted on the upper surface of the cushion body (64) to pass through the solution and filter the cells;
wherein a pressing step (56) formed on the inner circumferential surface of the lower coupling part (52) to press the edges of the membrane filter (63) and the cushion body (64), a locking step (55) formed on the inner circumferential surface of the lower coupling part (52) disposed between the bottom plate (53) and the pressing step (56), and a blocking step (57) formed on the inner circumferential surface of the lower coupling part (52) between the pressing step (56) and the locking step (55) are provided,
a vortex prevention net (70) whose edge is attached to the locking step (55) and through which the solution and cells pass is provided,
so that a first space (58) between the bottom plate (53) and the vortex prevention net (70), and a second space (59) between the vortex prevention net (70) and the membrane filter (63) are formed.

4. The container assembly for picking a free medical treatment formed pipet according to Claim 3, wherein a filter member (130) for filtering floating matter in the solution is further provided inside the cap member (120).
